# EUROPÄISCHE PATENTSCHRIFT

(11) **EP 3 646 334 B1**
(45) Veröffentlichungstag und Bekanntmachung des Hinweises auf die Patenterteilung: **28.08.2024**
(21) Anmeldenummer: 18743090.5
(22) Anmeldetag: 29.06.2018
(51) Int. Cl.: G16H 20/40, A61B 5/0536, A61B 5/08, A61B 5/00, A61M 16/00, G06F 3/0354, G06F 3/04847

(54) **BEATMUNGSGERÄT SOWIE VERFAHREN ZUM STEUERN EINES BEATMUNGSGERÄTS**
BREATHING APPARATUS AND METHOD FOR CONTROLLING A BREATHING APPARATUS
APPAREIL DE VENTILATION ET PROCÉDÉ DE COMMANDE D'UN APPAREIL DE VENTILATION

(30) Priorität: 30.06.2017 CH 8572017
(43) Veröffentlichungstag der Anmeldung: 06.05.2020
(73) Patentinhaber: imtmedical ag, 9470 Buchs (CH)
(72) Erfinder: FRIBERG, Harri, 9493 Mauren (LI); DÄSCHER, Jakob, 7306 Fläsch (CH)
(74) Vertreter: PPR AG
(86) Internationale Anmeldenummer: PCT/IB2018/054852
(87) Internationale Veröffentlichungsnummer: WO 2019/003197

(56) Entgegenhaltungen:
- CH-B1- 711 838
- US-A1- 2007 199 566
- US-A1- 2012 055 479

## Beschreibung

Die Erfindung betrifft ein Beatmungsgerät nach Anspruch 1.

Beatmungsgeräte werden sowohl im stationären (z. B. im Klinik- oder Heimbereich) als auch im mobilen Bereich (z. B. Rettungsdienst) eingesetzt. Dabei ist es wichtig, dass die Beatmungsgeräte zuverlässig und störungsfrei arbeiten.

Ein weiteres Bedürfnis an solche Beatmungsgeräte ist eine einfache Bedienbarkeit. Eine Fehlbedienung durch einen Bediener kann für den durch das Beatmungsgerät beatmete Patienten verheerende Folgen haben.

Die WO 02/071933 A2 offenbart ein Beatmungsgerät, das mit einer Sensorik sowie mit einer Steuerung verbunden ist, wobei die Steuerung mit einer Anzeigeeinrichtung verbunden ist. Die Sensorik erfasst Messdaten und überträgt diese an das Beatmungsgerät. Die Steuerung stellt Anzeigedaten auf Basis der erfassten Messdaten bereit, welche auf der Anzeigeeinrichtung als animierte Grafikeinheit einblendbar sind.

Die EP 1 984 805 B1 offenbart ein Beatmungsgerät, bei dem auf einer Anzeigeeinrichtung ein Grafikelement in Form einer Lunge dargestellt. Die Volumenänderung der beatmeten Lunge, welche bei jedem Atemzug auftritt, wird als eine animierte Grössenänderung der Lungenform darstellt. Eine vergleichbare Offenbarung ist in der CH 711 838 B1 gezeigt.

Die US 2007/0199566 A1 offenbart ein Beatmungsgerät, bei dem auf einer Anzeigeeinrichtung ein Grafikelement in Form einer Lunge dargestellt, wobei ein Luftaustritt und/oder Sekretaustritt animiert dargestellt wird.

Aufgabe der vorliegenden Erfindung ist es somit, ein Beatmungsgerät zu schaffen, das insbesondere für den Bediener einfach bedienbar und sicher in der Anwendung ist. Die erfassten Messdaten sollen dabei in qualitativer sowie quantitativer Hinsicht dem Bediener optimal zur Verfügung stehen.

Die Aufgabe wird durch die Merkmale des unabhängigen Anspruchs gelöst. Vorteilhafte Weiterbildungen sind in den Figuren und in den abhängigen Patentansprüchen dargelegt.

Nachfolgend wird in der Anmeldung der zwei Begriffe verbindende Ausdruck "oder" im Sinne von "und/oder" verwendet. Somit kann der erste Begriff oder der zweite Begriff für sich sowie aber zusammen in einer und-Verbindung verstanden werden. Das erfindungsgemässe Beatmungsgerät ist mit einer Sensorik sowie mit einer Steuerung verbunden. Die Steuerung kann Bestandteil des Beatmungsgerätes sein. Auch die Sensorik kann Bestandteil des Beatmungsgerätes sein. Die Sensorik ist zur Erfassung von zumindest zwei Messdaten sowie zum Übertragen der erfassten Messdaten an das Beatmungsgerät oder das Steuerlogikmodul ausgebildet.

Die Sensorik umfasst beispielsweise zumindest zwei Messsensoren, wobei jeder Messsensor Messdaten eines Ursprungs erfasst. Die Sensoren sind vorteilhaft unterschiedlich ausgebildet und erfassen verschiedene Messdaten. Alternativ umfasst die Sensorik nur einen Messsensor, der zumindest zwei Messdaten unterschiedlichen Ursprungs erfasst.

Die Steuerung ist mit zumindest einer Anzeigeeinrichtung verbunden, welche einen konfiguralen Bildschirm umfasst. Unter einem konfiguralen Bildschirm wird in diesem Zusammenhang ein Bildschirm, der nicht nur die Wahrnehmung der dargestellten Einzelkomponenten, sondern die Wahrnehmung der Gesamtheit aller dargestellten Komponenten und ihre Anordnung zueinander ermöglicht. Dabei erfasst der konfigurale Bildschirm entweder selbständig oder mithilfe eines Grafiklogikmoduls oder eines Steuerlogikmoduls Messdaten und wandelt diese in geometrische Elemente um, welche anschliessend eingeblendet werden können. Darüber hinaus ist der konfigurale Bildschirm geeignet, um bestehende Elemente (geometrische und/oder graphische) in einer Grafikeinheit zu verändern und Veränderungen an der Grafikeinheit in Parameter umzuwandeln, welche wiederum zum Steuern einer Steuerung herangezogen werden können.

Die Steuerung ist zur Bereitstellung von Anzeigedaten auf Basis der erfassten Messdaten ausgebildet, welche auf einer ersten Grafikeinheit auf der zumindest einen Anzeigeeinrichtung einblendbar sind, wobei zumindest einzelne Anzeigedaten in einer ersten animierbaren Darstellung eines Beatmungsgases auf der ersten Grafikeinheit der zumindest einen Anzeigeeinrichtung einblendbar sind und wobei einzelne Bestandteile des Beatmungsgases mit untereinander unterscheidbaren geometrischen Elemente darstellbar sind. Die erste Grafikeinheit ist eine bildliche Darstellung einer Lunge oder eines anderen, von dem Beatmungsgerät beeinflussten Organs. Weiter vorteilhaft umfasst die erste Grafikeinheit eine animierte Darstellung.

Durch den erfindungsgemässen Aufbau und die Verbindung der Elemente steht dem Bediener ein zuverlässiges und einfach bedienbares Beatmungsgerät zur Verfügung. Die visuelle Wahrnehmbarkeit ist für den Bediener durch den konfiguralen Bildschirm gegenüber den bekannten Beatmungsgeräten massgeblich verbessert. Dabei ist eine qualitative wie auch quantitative Wahrnehmung durch den Bediener am Beatmungsgerät jederzeit gewährleistet.

Vorzugsweise weist die Steuerung ein Steuerlogikmodul oder ein Grafiklogikmodul auf, womit die erfassten Messdaten einerseits in dem Steuerlogikmodul oder in dem Grafiklogikmodul oder sowohl in dem Steuerlogikmodul als auch in dem Grafiklogikmodul verarbeitet werden. Weiter können das Steuerlogikmodul und das Grafiklogikmodul eine gemeinsame Einheit bilden, die beispielsweise in dem Beatmungsgerät integriert ist. Weiter kann zumindest das Steuerlogikmodul auch der Steuerung des Beatmungsgeräts dienen.

All die vorgenannten Massnahmen für sich gewährleisten einen stabilen Betrieb des Beatmungsgerätes, womit eine hohe Ausfallsicherheit in der Verwendung desselben gegeben ist.

Vorteilhaft weisen das Steuerlogikmodul als auch das Grafiklogikmodul jeweils eine Recheneinheit auf, so dass in jedem Modul die erfassten Daten verarbeitet und zur weiteren Verwendung bereitgestellt werden können.

Bevorzugt ist der konfigurale Bildschirm ein berührungsempfindlicher Bildschirm, womit dieser nicht nur der Ausgabe von Anzeigedaten, sondern auch als Eingabegerät dienen kann. Derartige berührungsempfindliche Bildschirme werden auch Touchscreen genannt. Weitere, nicht abschliessende Beispiele für berührungsempfindliche Bildschirme stellen Touchpads oder Smartphones, SmartWatches dar, welche direkt oder indirekt, z. B. über eine drahtlose Verbindung, wie z. B. Bluetooth^{®}, WLAN, mit dem Beatmungsgerät oder Teilen davon verbunden sind.

Vorzugsweise ist ein Sensor zur Erfassung zumindest eines Bereichs der zumindest einen Anzeigeeinrichtung vorgesehen, womit eine nicht erwartete Veränderung der Anzeige einfach erfassbar und gegebenenfalls entsprechende Massnahmen, wie Warnmeldungen, interne Geräteprüfungen, gesetzt werden können. Fällt beispielsweise die Anzeige aus, kann dem Benutzer eine Meldung - beispielsweise auf seinen Pager oder sein Mobilphone - gesandt werden, so dass er zeitnah reagieren kann.

Weiter kann der Sensor visuell Anzeigen überwachen und so neben einer Überwachung durch die Steuerung eine zusätzliche, unabhängige Überwachungseinheit bilden. Damit wird die Sicherheit des Beatmungsgerätes zusätzlich erhöht.

Vorteilhaft ist dieser Sensor benachbart zu und weiter vorteilhaft direkt an der zumindest einen Anzeigeeinrichtung angeordnet, womit eine einfache konstruktive Ausgestaltung ermöglicht wird. Dieser Sensor kann Bestandteil der mit dem Beatmungsgerät verbundenen Sensorik sein.

Ein Verfahren zum Steuern des vorgenannten Beatmungsgeräts ist durch folgende Schritte gekennzeichnet:
Erfassen von zumindest zwei Messdaten mit der Sensorik (Schritt a)) und anschliessendes Übertragen der erfassten Messdaten von der Sensorik an das Beatmungsgerät oder an die Steuerung (Schritt b)).

Daraufhin erfolgt das Empfangen zumindest einzelner der erfassten Messdaten von dem Beatmungsgerät oder von der Steuerung (Schritt c)).

Vorteilhaft werden anschliessend einzelne der erfassten Messdaten von dem Beatmungsgerät oder von der Steuerung verarbeitet.

Anschliessend erfolgt das Bereitstellen von Anzeigedaten, welche auf Basis zumindest einzelner empfangener Messdaten erzeugt werden (Schritt d)).

Danach erfolgt das Einblenden zumindest einzelner Anzeigedaten in einer ersten animierbaren Darstellung eines Beatmungsgases auf einer ersten Grafikeinheit der zumindest einen Anzeigeeinrichtung, wobei zumindest einzelne Anzeigedaten in einer ersten animierbaren Darstellung eines Beatmungsgases auf der ersten Grafikeinheit der zumindest einen Anzeigeeinrichtung einblendbar sind und wobei einzelne Bestandteile des Beatmungsgases mit untereinander unterscheidbaren geometrischen Elemente darstellbar sind, (Schritt e)), womit die Anzeigedaten einfach von einem Bediener visuell und intuitiv wahrnehmbar sind.

Damit wird ein Verfahren zum Steuern eines Beatmungsgeräts beschrieben, welches eine hohe Ausfallsicherheit aufweist. Dem Bediener des Beatmungsgeräts (insbesondere dem medizinischen Fachpersonal) wird zumindest visuell auf Änderungen von Beatmungsparametern im Beatmungsgerät aufmerksam gemacht, womit dieser anschliessend so reagieren kann, dass der durch das Beatmungsgerät beatmende Patient keinen Schaden nimmt.

Die einzelnen Anzeigedaten können erfasste Messdaten, empfangene Messdaten oder verarbeitete Messdaten oder eine beliebige Kombination von erfassten Messdaten, empfangene und verarbeitete Messdaten beinhalten. Der Begriff verarbeitete Messdaten umfasst jede mathematische oder logische Veränderung der erfassten Messdaten. Die erfassten Messdaten werden von der Sensorik erfasst. Alternativ oder ergänzend dazu werden die erfassten Messdaten vom Bediener des Beatmungsgeräts an einer Eingabeeinrichtung eingegeben.

Die zumindest einzelnen Anzeigedaten werden vorteilhaft durch zumindest einzelne geometrische Elemente repräsentiert, womit die visuelle Wahrnehmbarkeit durch den Bediener zusätzlich vereinfacht und der Bediener visuell auf die einzelnen Anzeigedaten sensibilisiert wird.

Dabei werden vorteilhaft jene einzelnen Anzeigedaten, welche denselben Beatmungsparameter beschreiben, mit geometrischen Elementen gleicher geometrischen Eigenschaften eingeblendet und jene einzelnen Anzeigedaten, welche unterschiedliche Beatmungsparameter beschreiben, mit geometrischen Elementen unterschiedlicher geometrischen Eigenschaften eingeblendet.

Unter geometrischer Eigenschaft eines geometrischen Elements ist dessen Elementform, Elementfarbe und Elementgrösse zu verstehen. Dabei ist unter der Elementform eine zweidimensionale Form (Kreis, Dreieck, Ellipse, Polygon, usw.) oder eine dreidimensionale Form (Kugel, Pyramide, Kegel, Quader, usw.) zu verstehen.

Vorteilhaft werden die Anzeigedaten im Schritt d) durch die Steuerung bereitgestellt, womit die Anzeigedaten einfach bereitgestellt werden können. Beispielsweise werden die Anzeigedaten in dem Steuerlogikmodul breitgestellt.

Alternativ oder ergänzend werden Anzeigedaten durch ein Grafiklogikmodul bereitgestellt, womit neben einer graphischen Anzeige der Anzeigedaten auch eine einfache Anzeige der Anzeigedaten erfolgt und somit der Bediener Störfälle im Beatmungsgerät schnell erkennen sowie auf diese schnell reagieren kann.

Vorteilhaft ist das Grafiklogikmodul ein Bestandteil der Anzeigeeinrichtung, womit ein vereinfachter Aufbau im Beatmungsgerät gewährleistet ist.

Vorzugsweise werden die empfangenen Messdaten (Schritt c)) von dem Steuerlogikmodul der Steuerung an das Grafiklogikmodul übertragen, womit die Messdaten an der zumindest einen Anzeigeeinrichtung einfach graphisch eingeblendet werden können.

Vorteilhaft werden zumindest einzelne übertragene Messdaten von dem Grafiklogikmodul zur Bereitstellung von Anzeigedaten, verarbeitet. Mit dieser Massnahme kann die zu verarbeitende Messdatenmenge verringert werden.

Bevorzugt werden im Schritt c) die empfangenen Messdaten in der Steuerung in Messdatenklassen eingeteilt, wobei zumindest einzelne Messdaten zumindest einer Messdatenklasse an das Grafiklogikmodul übertragen werden. Damit wird die Messdatenmenge verringert, welche von der Grafiklogikeinrichtung verarbeitet werden muss.

Vorteilhaft werden alle Messdaten der zumindest einen Messdatenklasse an das Grafiklogikmodul übertragen, womit eine verbesserte Statistik in der anschliessend zu verarbeiteten Messdatenmenge gewährleistet ist.

Alternativ oder ergänzend werden zumindest einzelne Messdaten einer Messdatenklasse an die zumindest eine Anzeigeeinrichtung übertragen, womit beispielsweise ein fehlerhaftes Einblenden von Anzeigedaten verhindert wird.

Vorteilhaft werden alle Messdaten der einen Messdatenklasse an die zumindest eine Anzeigeeinrichtung übertragen, womit eine verbesserte Statistik in den angezeigten Anzeigedaten gewährleistet ist.

Vorzugsweise werden zumindest einzelne der Anzeigedaten mit einer weiteren animierbaren Darstellung in der zumindest einen Anzeigeeinrichtung eingeblendet, womit eine verbesserte visuelle Aufnahmefähigkeit des Bedieners des Beatmungsgeräts auf bestimmte Beatmungsparameter an der Anzeigeeinrichtung gewährleistet ist und dieser einfacher die notwendigen und insbesondere die richtigen Entscheidungen treffen kann. Somit können neben der ersten Grafikeinheit weitere animierte Einblendungen angezeigt werden, welche ebenfalls einfach von dem Bediener visuell wahrnehmbar sind.

Vorteilhaft werden zumindest einzelne der Anzeigedaten mit zumindest weiterer einzelner geometrischen Elementen in der weiteren animierbaren Darstellung in der zumindest einen Anzeigeeinrichtung eingeblendet, womit die visuelle Unterscheidbarkeit der einzelnen Anzeigedaten und somit der einzelnen Beatmungsparameter durch den Bediener des Beatmungsgerätes gefördert wird.

Vorteilhaft verändert sich zumindest eine geometrische Eigenschaft eines einzelnen geometrischen Elements in einer der animierbaren Darstellungen, sodass der Bediener des Beatmungsgeräts einen zeitlichen Verlauf der einzelnen Anzeigedaten und somit der einzelnen Beatmungsparameter beobachten kann. Damit kann der Bediener des Beatmungsgeräts auf Änderungen einfach und schnell reagieren. Weiter ist eine erhöhte Ausfallsicherheit im Beatmungsgerät gegeben.

Vorzugsweise werden zumindest einzelne der Anzeigedaten mit der weiteren animierbaren Darstellung in der ersten Grafikeinheit der zumindest einen Anzeigeeinrichtung eingeblendet, womit die visuelle Unterscheidbarkeit der einzelnen Anzeigedaten durch den Bediener des Beatmungsgerätes weiter verbessert wird.

Bevorzugt berechnet zumindest eine Recheneinheit des Steuerlogikmoduls oder des Grafiklogikmoduls mithilfe der empfangenen, einzelnen Messdaten zumindest eine Verteilung des Beatmungsgases. Damit werden fehlerhafte Messdaten statistisch eliminiert und somit ein verbesserter Messdatensatz generiert.

Alternativ oder ergänzend wird neben der berechneten Verteilung des Beatmungsgases, mithilfe der empfangenen, einzelnen Messdaten eine Anordnung des Beatmungsgases berechnet, womit neben einer statistischen Auswertung der Messdaten auch eine für den Bediener des Beatmungsgeräts bekannte Anordnungen des Beatmungsgases berechnet werden kann.

Vorzugsweise wird die Verteilung des Beatmungsgases zumindest in der ersten animierbaren Darstellung des Beatmungsgases eingeblendet, womit der Bediener des Beatmungsgeräts schnell auf eine Störung bei dem Beatmungsvorgang oder im Beatmungsgerät aufmerksam wird.

Alternativ oder ergänzend wird die Anordnung des Beatmungsgases zumindest in der ersten animierbaren Darstellung des Beatmungsgases eingeblendet, womit der Bediener des Beatmungsgeräts einfach auf eine Störung im Beatmungsgerät aufmerksam wird.

Vorteilhaft wird die animierbare Darstellung des Beatmungsgases mithilfe dem zumindest einen geometrischen Element eingeblendet, womit der auf einzelne geometrische Elemente geschulte Bediener des Beatmungsgeräts schnell reagieren kann.

Bevorzugt ist zumindest die erste Grafikeinheit der zumindest einen Anzeigeeinrichtung zumindest bereichsweise veränderbar, womit beispielsweise der Bediener aktiv in die Grafikeinheit eingreifen kann.

Vorteilhaft generiert eine Veränderung zumindest eines Bereichs der ersten Grafikeinheit einen Steuerwert, welcher anschliessend an die Steuerung übermittelt wird. Mit dieser Massnahme kann der Bediener des Beatmungsgeräts über die zumindest eine Grafikeinheit direkt in die Steuerung eingreifen, was eine einfache Bedienung des Beatmungsgeräts aber auch eine hohe Ausfallstabilität gewährleistet.

Vorzugsweise ist die zumindest eine erste animierbare Darstellung eines Beatmungsgases in der zumindest einen ersten Grafikeinheit der Anzeigeeinrichtung abgebildet, wobei die zumindest eine erste animierbare Darstellung des Beatmungsgases mithilfe zumindest einzelner Anzeigedaten repräsentiert wird. Damit wird eine verbesserte visuelle Sensibilisierung des Bedieners des Beatmungsgeräts auf die Anzeigedaten gewährleistet.

Vorzugsweise beschreibt das zumindest einzelne eingeblendete geometrische Element, welches zumindest einzelne Anzeigedaten repräsentiert, zumindest einen Beatmungsparameter, womit der Bediener vorab visuell auf jedes einzelne, geometrische Element geschult werden kann und den zumindest einen Beatmungsparameter dem geometrischen Element zuordnen kann.

Vorteilhaft beschreibt das zumindest einzelne eingeblendete geometrische Element zumindest einen Beatmungsparameter aus der Gruppe der Sauerstoffparameter, Kohlendioxidparameter und Lungendruckparameter, wobei zumindest einzelne eingeblendete geometrische Element mit zumindest einer eigenen geometrischen Eigenschaft repräsentiert wird. Mit dieser Massnahme wird ein visuelles Anzeigen der Beatmungsparameter an der zumindest einen Anzeigeeinrichtung ermöglicht.

Bevorzugt ist in der zumindest einen ersten animierbaren Darstellung des Beatmungsgases, ein verbrauchter Beatmungsgasanteil von einem unverbrauchten Beatmungsgasanteil unterscheidbar, wobei die jeweiligen Beatmungsgasanteile mit unterschiedlichen einzelnen geometrischen Eigenschaften eingeblendet werden. Damit erhält der Bediener des Beatmungsgeräts eine schnellen Überblick kann schnell auf Störungen im Beatmungsgerät reagieren.

Vorzugsweise sind Messdaten einzelner Beatmungsparameter animierbar eingeblendet, womit der Bediener einfach auf eine etwaige Fehlfunktion im Beatmungsgerät reagieren kann.

Alternativ oder ergänzend sind Differenzwerte von Messdaten verschiedener Beatmungsparameter animierbar eingeblendet, womit zusätzlich verschiedene Beatmungsparameter am Beatmungsgerät verändert werden können.

Bevorzugt ist zumindest eine weitere animierbare Darstellung in der Anzeigeeinrichtung vorgesehen, welche zumindest einen Teil der ersten Grafikeinheit umfasst, wobei der zumindest eine Teil der ersten Grafikeinheit mit dessen geometrischen Eigenschaften hervorgehoben ist. Damit wird die visuelle Wahrnehmbarkeit des Bedieners des Beatmungsgeräts auf einzelne besonders wichtige Beatmungsparameter sensibilisiert.

Vorteilhaft wird der zumindest eine Teil der ersten Grafikeinheit bereichsweise mit dessen geometrischen Eigenschaften hervorgehoben, womit der Bediener des Beatmungsgerät auf einen wichtigen Bereich am zumindest einen Teile der Grafikeinheit hingeführt wird.

Vorzugsweise weist die zumindest eine Anzeigeeinrichtung eine weitere Grafikeinheit auf, welche ein Diagramm mit einem graphischen Element, z. B. eine Linie, aufweist, wobei das graphische Element zumindest einen zeitlichen Verlauf von Anzeigedaten repräsentiert, wobei die Anzeigedaten zumindest einen Beatmungsparameter darstellen. Damit ist die zeitliche Entwicklung eines Beatmungsparameters rückschauend beobachtbar.

Vorteilhaft ist das graphische Element im Diagramm mit zumindest einer geometrischen Eigenschaft des entsprechenden geometrischen Elements in einer der animierbaren Darstellungen abgestimmt, womit die Orientierung des Bedieners des Beatmungsgeräts an der zumindest einen Anzeigeeinrichtung verbessert wird.

Bevorzugt weist die weitere Grafikeinheit ein Balkendiagramm zum animierbaren Darstellen eines Parameters des Beatmungsgeräts auf. Damit wird dem Benutzer ein besonders relevanten Parameter graphisch präsentiert.

Insbesondere weist das Balkendiagramm eine obere Schranke und eine untere Schranke auf, womit typischerweise ein maximal erlaubter Wert bzw. minimal erlaubter Wert des Parameters darstellbar ist und somit für den Benutzer ein Risikobereich des Parameters darstellbar ist.

Bevorzugt ist das Beatmungsgerät mit einer weiteren Anzeigeeinrichtung verbunden, welche einzelne Anzeigedaten vom Beatmungsgerät erhält, wobei die zumindest eine Anzeigeeinrichtung und die weitere Anzeigeeinrichtung vorteilhaft räumlich getrennt sind, womit der Bediener des Beatmungsgeräts Information zum Beatmungsgerät von verschiedenen Geräten erhält und auch über eine Distanz schnell reagieren kann.

Vorzugsweise ist das Beatmungsgerät mit einer Tomographiemessvorrichtung verbunden, wobei zumindest einzelne Messdaten der Tomographiemessvorrichtung an die Steuerung übertragen und zumindest in der Steuerung empfangen werden, welche in eine der animierbaren Darstellungen in der ersten Grafikeinheit berücksichtigbar sind. Damit ist eine verbesserte Berechnung der Verteilung oder der Anordnung des Beatmungsgases in der ersten Grafikeinheit möglich.

Vorteilhaft ist die Tomographiemessvorrichtung eine elektrischen Impedanztomographiemessvorrichtung, womit eine besonders genaue Bestimmung von Messdaten für das Beatmungsgerät erfolgt und eine besonders genaue Berechnung der Verteilung oder der Anordnung des Beatmungsgases in der ersten Grafikeinheit möglich ist.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei:
- Fig. 1: Eine erste Ausführungsform des Beatmungsgeräts mit einer ersten erfindungsgemässen animierbaren Darstellung eines Beatmungsgases in einer Lunge als erste Grafikeinheit auf einer Anzeigeeinrichtung in einer perspektivischen Darstellung,
- Fig. 2: zeigt die animierbare Darstellung des Beatmungsgases in einer Lunge als erste Grafikeinheit gemäss Fig. 1 beim Einatmen in einer perspektivischen Darstellung,
- Fig. 3: zeigt die animierbare Darstellung des Beatmungsgases in einer gefüllten Lunge als erste Grafikeinheit gemäss Fig. 1 in einer perspektivischen Darstellung,
- Fig. 4: zeigt eine weitere animierbare Darstellung des Beatmungsgases in einer Lunge als erste Grafikeinheit gemäss Fig. 1 in einer perspektivischen Darstellung,
- Fig. 5: zeigt eine weitere animierbare Darstellung des Beatmungsgases in einer Lunge als erste Grafikeinheit gemäss Fig. 1 in einer weiteren perspektivischen Darstellung,
- Fig. 6: zeigt eine weitere animierbare Darstellung des Beatmungsgases in einer Lunge als erste Grafikeinheit gemäss Fig. 1 in einer weiteren perspektivischen Darstellung,
- Fig. 7: zeigt eine weitere animierbare Darstellung des Beatmungsgases in einer Lunge als erste Grafikeinheit gemäss Fig. 1 in einer weiteren perspektivischen Darstellung,
- Fig. 8: zeigt eine weitere animierbare Darstellung des Beatmungsgases in einer Lunge als erste Grafikeinheit gemäss Fig. 1 in einer weiteren perspektivischen Darstellung,
- Fig. 9: zeigt eine weitere animierbare Darstellung des Beatmungsgases in einer Lunge als erste Grafikeinheit gemäss Fig. 1 in einer weiteren perspektivischen Darstellung,
- Fig. 10: zeigt eine weitere animierbare Darstellung des Beatmungsgases in einer Lunge als erste Grafikeinheit gemäss Fig. 1 in einer weiteren perspektivischen Darstellung, und
- Fig. 11: zeigt eine weitere animierbare Darstellung des Beatmungsgases in einer Lunge als erste Grafikeinheit gemäss Fig. 1 in einer weiteren perspektivischen Darstellung.
- Fig. 12: zeigt eine weitere animierbare Darstellung in einem Balkendiagramm als zweite Grafikeinheit gemäss Fig. 1 in einer perspektivischen Darstellung

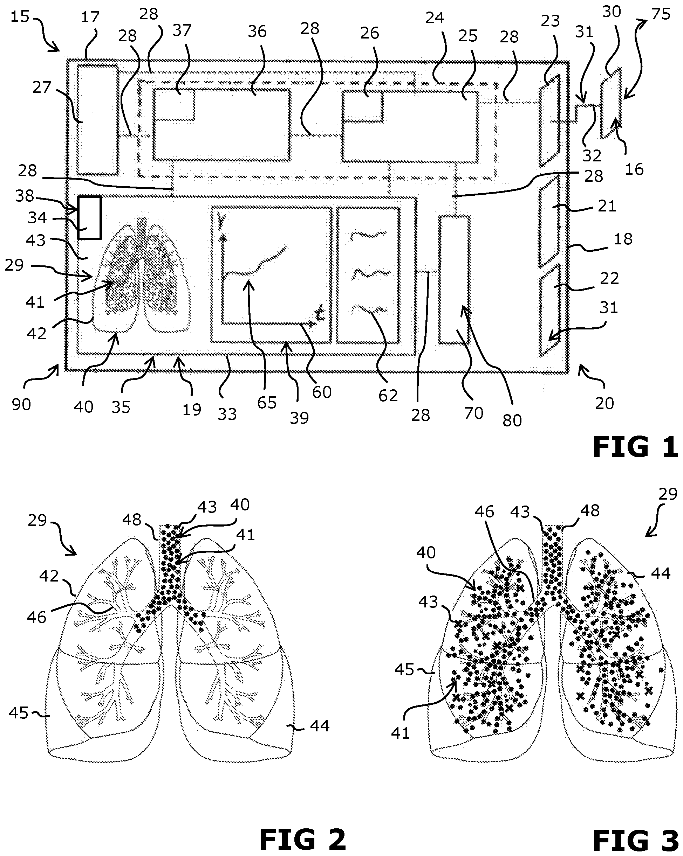

Die Fig. 1 zeigt ein Beatmungsgerät 15 mit einem Gehäuse 17, an welchem an dessen Gehäusewandung 18 eine Anschlusseinrichtung 20 angeordnet ist. An der Gehäusefront 19 ist eine erste Anzeigeeinrichtung 35 angeordnet. Im Gehäuse 17 des Beatmungsgeräts 15 befindet sich eine Steuerung mit einem Steuerlogikmodul 25, das eine Recheneinheit 26 (bspw. einen Prozessor) und eine Speichereinrichtung 27 aufweist, sowie mit einem Grafiklogikmodul 36, das eine Recheneinheit 37 (bspw. einen Prozessor) aufweist. Das Steuerlogikmodul 25 und das Grafiklogikmodul 36 sind miteinander mithilfe Datenleitungen 28 elektrisch verbunden. Die Anschlusseinrichtung 20 umfasst neben Versorgungsanschlüssen 21 (wie Stromversorgung, Internetanschluss, Gatewayanschluss usw.) einen Beatmungsschlauchanschluss 22 sowie mehrere Sensoranschlüsse 23. Mithilfe der Sensoranschlüsse 23 und des Beatmungsschlauchanschlusses 22 werden erfasste Messdaten 31 von der externen Sensorik 30 mithilfe herkömmlicher Datenverbindungen 32 (Kabel, WLAN, Bluetooth^{®}, usw.) und beispielsweise A/D-Wandler (nicht gezeigt) an das Steuerlogikmodul 25 übertragen. Dort werden die erfassten Messdaten 31 entweder direkt verarbeitet und/oder an das Grafiklogikmodul 36 übertragen sowie zumindest teilweise in der Speichereinrichtung 27 gespeichert. Das Steuerlogikmodul 25 ist mit der ersten Anzeigeeinrichtung 35 via Datenleitungen 28 verbunden. An der ersten Anzeigeeinrichtung 35 ist ein Sensor 34 vorgesehen, welcher einen Bereich 38 der Anzeigeeinrichtung 35 erfasst. Die erste Anzeigeeinrichtung 35 weist einen konfiguralen Bildschirm 33 mit eine erste Grafikeinheit 29 und eine zweite Grafikeinheit 39 auf. Die erste Grafikeinheit 29 umfasst die animierbare Darstellung 40 des Beatmungsgases 41 in der Lunge 42. Der zweite Grafikeinheit 39 zeigt ein Diagramm 60 (y,t Diagramm) mit dem der zeitliche Verlauf eines der Anzeigedaten 65 sowie die numerischen Angaben einzelner Anzeigedaten 62 eingeblendet werden. Die Beatmungsparameter 16 werden mithilfe der Anzeigedaten 62, 65 direkt an der erste Anzeigeeinrichtung 35 eingeblendet oder werden vorab in der Recheneinheit 26 des Steuerlogikmoduls 25 verarbeitet und anschliessend als Anzeigedaten 65 an der erste Anzeigeeinrichtung 35 und/oder in der ersten Grafikeinheit 29 mit einer entsprechenden Verteilung des Beatmungsgases 41 (homogenen oder inhomogenen Verteilung, Gaussverteilung, Exponentialverteilung, usw.) eingeblendet. Die Gehäusefront 19 weist des Weiteren eine Eingabeeinrichtung 70 auf, welche mithilfe Datenleitungen 28 mit dem Steuerlogikmodul 25 elektrisch verbunden ist und mit welcher der Bediener 90 (bspw. medizinisches Fachpersonal) des Beatmungsgeräts 15 einzelne Beatmungsparameter 16 sowie Patientenparameter 80 eingibt.

Das Beatmungsgerät 15 ist beispielsweise mit einer Tomographiemessvorrichtung (nicht gezeigt) verbunden, welche deren Messdaten 31 an das Steuerlogikmodul 25 überträgt. Diese Messdaten 31 fliessen in die Verarbeitung von Beatmungsparametern 16 ein, wobei die Recheneinheit 26 des Steuerlogikmoduls 25 damit beispielsweise die Verteilung des Beatmungsgases 43 in der Lunge 42 berechnet und anschliessend das Ergebnis daraus in der animierbaren Darstellung 40 des Beatmungsgases 41 einfliessen lässt. Als bevorzugte Tomographiemessvorrichtung ist eine elektrische Impedanztomographiemesseinrichtung vorgesehen.

Die nachfolgenden Figuren 2 bis 11 zeigen die unterschiedlichen Ausführungsformen der animierbaren Darstellungen 40, 50 des Beatmungsgases 41 in der Lunge 42 in der ersten Grafikeinheit 29, wobei die Lunge 42 aus zwei Lungenabschnitten 44, 45 bzw. Lungenflügel besteht, welche mittels der Luftröhre 48 sowie den jeweiligen Bronchien 46 miteinander verbunden sind. Das Beatmungsgas 41 setzt sich aus einer Vielzahl von Bestandteilen (bspw. Sauerstoff, Stickstoff, Edelgase, Kohlendioxid, usw.) zusammen, welche mithilfe unterschiedlicher Beatmungsparameter 16 bzw. Anzeigedaten 62, 65 an der erste Anzeigeeinrichtung 35 angezeigt werden sowie mit untereinander unterscheidbaren geometrischen Elemente 43 dargestellt werden. Die geometrischen Elemente 43 werden dabei zweidimensional (bspw. Kreise, Striche, Dreiecke, usw.) bzw. dreidimensional (Kugeln, Balken, Pyramiden, usw.) angezeigt. Je nach Ausführungsform des erfinderischen Beatmungsgeräts 15 sind die geometrischen Elemente 43 der animierbaren Darstellung 40 in unterschiedlichen Elementgrösse, Elementform sowie Elementfarbe veränderbar eingeblendet.

Beispielsweise werden in der animierbaren Darstellung 40 alle Beatmungsparameter 16 bzw. Anzeigedaten 62, 65 als Kreise eingeblendet, welche sich in deren Durchmesser unterscheiden.

In der animierbaren Darstellung 40 verteilen sich die geometrischen Elemente 43 beim Füllen der Lunge 42 im gesunden Zustand ausgehend von der Luftröhre 48 über die Bronchien 46 in den beiden Lungenabschnitten 44, 45 homogen und vollständig (Fig. 2 und Fig. 3). Dabei sind der Parameter der Sauerstoffkonzentration, welcher von dem Steuerlogikmodul 25 mithilfe dem gemessenen eingeatmeten Saustoffs (FiO2_mess), dem festgelegten Bruchteil des eingeatmeten Saustoffs (FiO2_set) und der gemessenen Sauerstoffsättigung (SpO2) bereitgestellt wird, der Parameter des Kohlendioxids, welcher mithilfe eines CO2-Sensors gemessen wird, sowie der Parameter des Lungenüberdrucks, welcher von dem Steuerlogikmodul 25 mithilfe des gemessenen proximalen Drucks und des Tracheadrucks bereitgestellt wird, jeweils mit dem gleichen geometrischen Element 43, jedoch unterschiedlichen Elementfarbe und/oder Elementgrösse in der animierbaren Darstellung 40 unterscheidbar (Fig.3).

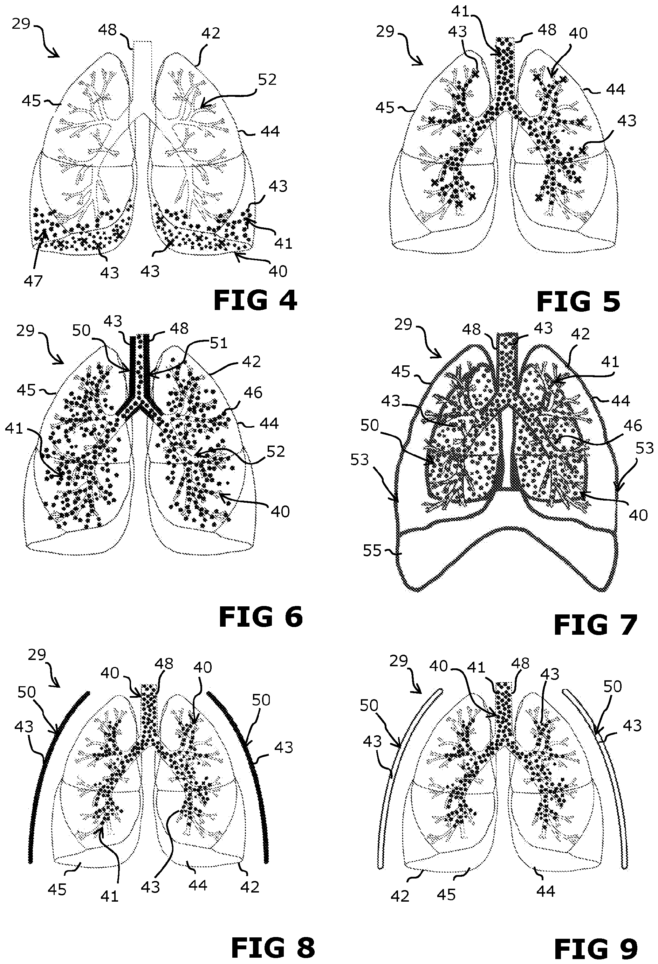

Figur 4 zeigt die animierbare Darstellung 40 des Beatmungsgases 41 in der Lunge 42, wobei sich das Beatmungsgas 41 bei einer hyperinflationären Lunge 42 im unteren Lungenbereich 47 der Lungenabschnitte 43, 44 ansammelt. Bei einer ständigen Messung des automatischen Positiven-Ausatemdruckparameters (Auto-PEEP) mithilfe geeigneter Sensorik 30, wird ein sich erhöhender Messwert des Auto-PEEPs von dem Steuerlogikmodul 25 ausgewertet und in der animierbaren Darstellung 40 dargestellt. Hierzu ist in der animierbaren Darstellung 40 der verbrauchte Beatmungsgasanteil (bspw. der gesättigte Kohlendioxidanteil bzw. der verbrauchte Sauerstoffanteil) und der unverbrauchte Beatmungsgasanteil (neu zugeführten Beatmungsgasen 41) in unterschiedlichen Graustufen mit dem gleichen geometrischen Element 43 darstellt.

Bei einer Messung des PEEPs sind mithilfe der animierbaren Darstellung 40 des Beatmungsgases 41 jene Lungenbereiche 47 (bspw. Lungenbläschen) darstellbar, welche noch Überreste des Beatmungsgases 41 aufweisen. Diese Lungenbläschen an den Bronchien 46 werden jeweils mithilfe eines geometrischen Elements 43 (Kreis) dargestellt (Fig.5).

Wie in Fig. 6 gezeigt ist mithilfe der weiteren Darstellung 50 eine Restriktion der Luftröhre 48 animierbar darstellbar. Dabei ist die Luftröhrenwand 51 sowie die Bronchienwand 52 verbreitert dargestellt und farblich von einer gesunden Lunge unterscheidbar dargestellt. Zusätzlich ist das Beatmungsgas 41 in dieser animierten Darstellung 40 so angeordnet, dass die geometrischen Elemente 43 nacheinander linear angeordnet sind.

Fig. 7 zeigt eine Lunge 42 mit einer erhöhten Lungenhärte. Diese wird mithilfe der Compliance als Beatmungsparameter 16 von dem Steuerlogikmodul 25 ermittelt und ist mittels einer Kombination der ersten animierbaren Darstellung 40 des Beatmungsgases 41, welche eine räumlich eingeschränkte Verteilung der geometrischen Elemente 43 in den Lungenabschnitten 44, 45 zeigt, und der weiteren animierbaren Darstellung 50 dargestellt, welche eine farbliche Hervorhebung der Lungenflügelwand 53 zeigt. Dabei lässt sich der Grad der Lungenflügelhärte mit der Breite der farblichen Hervorhebung der Lungenflügelwand 53 repräsentieren. Zusätzlich ist das Zwerchfell 55 in einer unterscheidbaren Farbe dargestellt, welches bei der Messung einer spontanen Atemanstrengung des Patienten in dem Steuerlogikmodul 25 verarbeitet wird und in der weiteren animierbaren Darstellung 50 gezeigt ist.

Fig. 8 und Fig. 9 zeigen die animierbare Darstellung 50 einer osophogalen Druckmessung in der Lunge 42, wobei die Rückschlüsse aus der osophogalen Druckmessung mithilfe der geometrischen Elemente 43 in Form von Messbalken ausserhalb der Lunge 42 angezeigt sind. Dabei verarbeitet das Steuerlogikmodul 25 Messdaten 31 der Lungendruckmessung und der Interpleuraldruckmessung an der Lunge 42, indem beispielsweise ein Differenzwert der Messdaten 31 gebildet wird, welcher anschliessend als Anzeigedaten 62, 65 in Messbalkenform mit verschiedenen Farben in der animierbaren Darstellung 50 dargestellt werden.

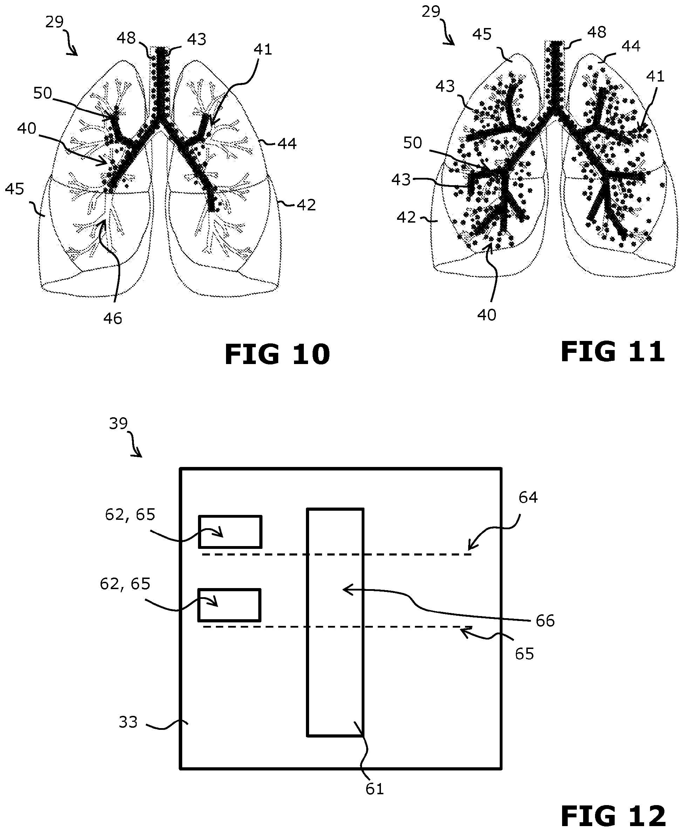

Fig. 10 und Fig. 11 zeigen die Darstellung eines Verhältniswertes des PEEP-Wertes zum Differenzwert des PEEP-Wertes und des Drucks beim Einatmen (PINSP), welcher von dem Steuerlogikmodul 25 verarbeitet wird und anschliessend mithilfe der animierbaren Darstellung 40 in der Lunge dargestellt wird. Bei erhöhtem Verhältniswerten werden die geometrischen Elemente 43 in deren Elementfarbe verändert und mithilfe des Messbalkens in der Lunge 42 farblich hervorgehoben. Das erfindungsgemässe Verfahren zum Steuern eines Beatmungsgeräts 15 umfasst in einer bevorzugten Ausführungsform folgende Schritte:
Nachdem die Sensorik 30 Messdaten 31 erfasst hat, werden die Messdaten 31 an das Beatmungsgerät 15 und dessen Steuerung 24 geliefert, welche anschliessend vom Beatmungsgerät 15 verarbeitet werden, indem die Messdaten 31 in der Speichereinrichtung 27 gespeichert werden und/oder werden in der Steuerung 24 verarbeitet. Dort werden Messdaten 31 entweder mit Daten aus der Speichereinrichtung 27 kombiniert oder so verarbeitet, sodass diese als Anzeigedaten 62, 65 eingeblendet werden. Im Verarbeitungsprozess kombiniert die Recheneinheit 26 des Steuerlogikmoduls 25 oder die Recheneinheit 37 des Grafiklogikmoduls 36 die Messdaten 31 (gegebenenfalls mit historischen Messdaten) von eingegangen Beatmungsparameter 16 quantitativ und qualitativ. Nach der Kombination der Beatmungsparameter 16 weist das Steuerlogikmodul 25 jene Beatmungsparameter 16, welche in einem der animierbaren Darstellungen 40, 50 des Beatmungsgases 41 dargestellt werden, jeweils ein geometrisches Element 43 zu und zeigt dieses mit zugeordneter Elementform, Elementfarbe und Elementgrösse in der ersten Grafikeinheit 29 an. Geleichzeitig bestimmt das Steuerlogikmodul 25 oder das Grafiklogikmodul 36 den zeitlichen Verlauf der gleichen Beatmungsparameter 16 und zeigt diese im Diagramm 60 mit den gleichen Farben bzw. mit der gleichen Form bzw. Elementgrösse an. Zeitgleich werden Anzeigeparameter 62 an der zweiten Grafikeinheit angezeigt.

Beispielsweise lässt sich damit das Aufreissen verklebter Lungenbereiche (Lungenrekrutierung) animiert darstellen. Dabei wird in einem ersten Schritt ein steuerbarer Beatmungsdruck (bspw. der PEEP) langsam erhöht, wobei dessen zeitlicher Verlauf im Diagramm 60 sowie das zugeordnete geometrische Element 43 in der animierbaren Darstellung 40 in der gleichen Farbe angezeigt werden. Anschliessend wird die Beatmung gestoppt, der Beatmungsdruck (bspw. der PEEP) langsam wieder reduziert, wobei dessen zeitlicher Verlauf im Diagramm 60 sowie das geometrische Element 43 in der animierbaren Darstellung 40 in der gleichen Farbe, jedoch unterscheidbar zum ersten Schritt, angezeigt werden. Diese beiden Schritte werden solange wiederholt, bis die grösste Differenz (die Hysterese) in den beiden Schritten feststeht. Der damit bestimmte Beatmungsdruck (bspw. der PEEP) wird in weiterer Folge von dem Steuerlogikmodul 25 an die Steuerung 24 weitergegeben und als neuer als Steuerwert im Beatmungsgerät 15 vorgegeben wird. Der Bediener 90 kann bei einer Veränderung (gegebenenfalls bei einer unvorhersehbaren Störung) am Beatmungsgerät 15 direkt in die Steuerung 24 eingreifen, indem er einen der Anzeigedaten 62, 65 in der ersten Grafikeinheit 29 verändert. Dabei wird ein Steuerwert generiert der anschliessend an die Steuerung 40 übermittelt wird. Die oben angeführten geometrischen Elemente 43, welche die einzelnen Beatmungsparameter 16 bzw. Anzeigeparameter 65 in der Lunge 42 repräsentieren, können sich je nach Ausführungsform in deren Form, Grösse sowie Farbe voneinander unterscheiden.

Fig 12 zeigen eine animierbare Darstellung eines Parameters 66 in einem Balkendiagramm 61 als zweite Grafikeinheit 39 am konfiguralen Bildschirm 33. Das Balkendiagramm 61 weist eine obere Schranke 64 und eine untere Schranke 65 auf. Beispielsweise stellt das Balkendiagramm 61 einen besonders relevanten Parameter 66 des Beatmungsgeräts dar, wie etwa die Beatmungsleistung, oder die Gesamtleistung oder die transpulmonale Leistung. Der maximal erlaubte Wert bzw. minimal erlaubte Wert des Parameters 66 sind durch die obere Schranke 64 bzw. durch die untere Schranke 65 dargestellt, wodurch für den Benutzer 90 der Risikobereich des Parameters 66 darstellbar ist. Gleichzeitig werden weitere für den Parameter 66 signifikante Anzeigedaten 62, 65, wie beispielsweise das Totvolumen oder die Beatmungsrate, an der oberen Schranke 64 und der unteren Schranke 65 dargestellt. Die obere Schranke 64 und die untere Schranke 65 sind auf den zu beatmeten Patienten 75 abstimmbar, wobei eine Veränderung des Parameters 66 animiert darstellbar ist. Beispielsweise ist eine Änderung des Parameters 66 mit einer animierten Änderung der Darstellungsfarbe darstellbar. Die Darstellung der zweiten Grafikeinheit 39 ist zusammen mit der Darstellung der ersten Graphikeinheit 29 animiert darstellbar.

### Bezugszeichenliste

- 15: Beatmungsgerät
- 16: Beatmungsparameter
- 17: Gehäuse
- 18: Gehäusewandung
- 19: Gehäusefront
- 20: Anschlusseinrichtung
- 21: Versorgungsanschlüsse
- 22: Beatmungsschlauchanschluss
- 23: Sensoranschlüsse
- 24: Steuerung
- 25: Steuerlogikmodul
- 26: Recheneinheit von 25
- 27: Speichereinrichtung
- 28: Datenleitungen
- 29: erste Grafikeinheit
- 30: Messdaten
- 31: Sensorik
- 32: Datenverbindung
- 33: konfiguraler Bildschirm
- 34: Sensor
- 35: erste Anzeigeeinrichtung
- 36: Grafiklogikmodul
- 37: Recheneinheit von 36
- 38: Bereich
- 39: zweite Grafikeinheit
- 40: animierbare Darstellung
- 41: Beatmungsgas
- 42: Lunge
- 43: geometrisches Element
- 44: Lungenabschnitt
- 45: Lungenabschnitt
- 46: Bronchien
- 47: Lungenbereich
- 48: Luftröhre
- 50: weitere animierbare Darstellung
- 51: Luftröhrenwand
- 52: Bronchienwand
- 53: Lungenflügelwand
- 55: Zwerchfell
- 60: Diagramm (y,t Diagramm)
- 61: Balkendiagramm
- 62: Anzeigedaten (numerisch)
- 63: untere Schranke
- 64: obere Schranke
- 65: Anzeigedaten (graphisch)
- 66: Parameter
- 70: Eingabeeinrichtung
- 75: Patient
- 80: Patientenparameter
- 90: Bediener

## Patentansprüche

1. Beatmungsgerät (15), das mit einer Sensorik (30) sowie mit einer Steuerung (24) verbunden ist, wobei
die Sensorik (30) zur Erfassung von zumindest zwei Messdaten (31) sowie zum Übertragen der erfassten Messdaten (31) an das Beatmungsgerät (15) oder das Steuerlogikmodul (25) ausgebildet ist, und wobei
die Steuerung (24) mit zumindest einer Anzeigeeinrichtung (35) verbunden ist,
**dadurch gekennzeichnet, dass**
die zumindest eine Anzeigeeinrichtung (35) einen konfiguralen Bildschirm (33) umfasst, und wobei
die Steuerung (24) zur Bereitstellung von Anzeigedaten (62, 65) auf Basis der erfassten Messdaten (31) ausgebildet ist, welche auf einer ersten Grafikeinheit (29) auf der zumindest einen Anzeigeeinrichtung (35) einblendbar sind, wobei zumindest einzelne Anzeigedaten (62, 65) in einer ersten animierbaren Darstellung (40) eines Beatmungsgases (41) auf der ersten Grafikeinheit (29) der zumindest einen Anzeigeeinrichtung (35) einblendbar sind, wobei die ersten Grafikeinheit (29) eine bildliche Darstellung einer Lunge (42) ist, und wobei einzelne Bestandteile des Beatmungsgases (41) mit untereinander unterscheidbaren geometrischen Elementen (43) darstellbar sind.

2. Beatmungsgerät nach Anspruch 1, **dadurch gekennzeichnet, dass** die Steuerung (24) ein Steuerlogikmodul (25) oder ein Grafiklogikmodul (36) aufweist, die vorteilhaft jeweils eine Recheneinheit (26, 37) aufweisen.

3. Beatmungsgerät nach Anspruch 1 oder 2, **dadurch gekennzeichnet, dass** der konfigurale Bildschirm (33) ein berührungsempfindlicher Bildschirm ist.

4. Beatmungsgerät nach einem der Ansprüche 1 bis 3, **dadurch gekennzeichnet, dass** ein Sensor (34) zur Erfassung zumindest eines Bereichs (38) der zumindest einen Anzeigeeinrichtung (35) vorgesehen ist.

5. Beatmungsgerät nach einem der Ansprüche 1 bis 4, **dadurch gekennzeichnet, dass** zumindest eine weitere animierbare Darstellung (50) in der Anzeigeeinrichtung (35) vorgesehen ist, welche zumindest einen Teil der ersten Grafikeinheit (29) umfasst, wobei der zumindest eine Teil der ersten Grafikeinheit (29) mit dessen geometrischen Eigenschaften hervorgehoben ist, wobei bevorzugt die weitere animierbare Darstellung (50) eine farbliche Hervorhebung der Lungenflügelwand (53) der Lunge darstellbar ist und/oder eine Restriktion der Luftröhre (48) animierbar darstellbar ist und/oder das Zwerchfell (55) in einer unterscheidbaren Farbe darstellbar ist.

## Claims

1. A ventilator (15) which is connected to a sensor system (30) as well as to a control system (24),
wherein
the sensor system (30) is configured to acquire at least two items of measurement data (31) as well as to transmit the acquired measurement data (31) to the ventilator (15) or to the control logic module (25), and wherein
the control system (24) is connected to at least one display device (35), **characterized in that**
the at least one display device (35) comprises a configural display screen (33), and wherein
the control system (24) is configured to provide display data (62, 65) on the basis of the acquired measurement data (31) which can be displayed on a first graphics unit (29) on the at least one display device (35), wherein at least individual items of display data (62, 65) can be displayed in a first animatable representation (40) of a respiratory gas (41) on the first graphics unit (29) of the at least one display device (35), wherein the first graphics unit (29) is a pictorial representation of a lung (42), and wherein individual components of the respiratory gas (41) can be represented by mutually distinguishable geometrical elements (43).

2. The ventilator as claimed in claim 1, **characterized in that** the control system (24) has a control logic module (25) or a graphic logic module (36) which advantageously each have a processing unit (26, 37).

3. The ventilator as claimed in claim 1 or claim 2, **characterized in that** the configurable screen (33) is a touch-sensitive screen.

4. The ventilator as claimed in one of claims 1 to 3, **characterized in that** a sensor (34) is provided for surveying at least one region (38) of the at least one display device (35).

5. The ventilator as claimed in one of claims 1 to 4, **characterized in that** at least one further animatable representation (50) which comprises at least a portion of the first graphics unit (29) is provided in the display device (35), wherein the at least a portion of the first graphics unit (29) with its geometric properties is highlighted, wherein preferably, the further animatable representation (50) can be represented by coloured highlighting of the wall of the lobe of the lung (53) and/or a restriction in the trachea (48) can be animated and/or the diaphragm (55) can be represented in a distinguishable colour.

## Revendications

1. Respirateur (15), qui est connecté avec un système de capteurs (30), ainsi qu'avec un dispositif de commande (24),
le système de capteurs (30) étant conçu pour détecter au moins deux données de mesure (31), ainsi que pour transmettre les données de mesure (31) détectées au respirateur (15) ou au module de logique de commande (25) et
le dispositif de commande (24) étant connecté avec au moins un système d'affichage (35),
**caractérisé en ce que**
l'au moins un système d'affichage (35) comprend un écran (33) de traitement configural,
et
le dispositif de commande (24) étant conçu pour la mise à disposition de données d'affichage (62, 65) sur la base des données de mesure (31) détectées, qui sont affichables sur une première unité graphique (29) sur l'au moins un système d'affichage (35), au moins des données d'affichage (62, 65) individuelles étant affichables dans une première représentation (40) susceptible d'être animée d'un gaz respiratoire (41) sur la première unité graphique (29) de l'au moins un système d'affichage (35), la première unité graphique (29) étant une représentation visuelle d'un poumon (42) et des composants individuels du gaz respiratoire (41) étant susceptibles d'être représentés par des éléments (43) géométriques distinguibles les uns des autres.

2. Respirateur selon la revendication 1, **caractérisé en ce que** le dispositif de commande (24) comporte un module de logique de commande (25) ou un module de logique graphique (36), qui avantageusement comportent chacun une unité de calcul (26, 37).

3. Respirateur selon la revendication 1 ou 2, **caractérisé en ce que** l'écran (33) de traitement configural est un écran tactile.

4. Respirateur selon l'une quelconque des revendications 1 à 3, **caractérisé en ce qu'**il est prévu un capteur (34), destiné à détecter au moins une région (38) de l'au moins un système d'affichage (35).

5. Respirateur selon l'une quelconque des revendications 1 à 4, **caractérisé en ce qu'**il est prévu au moins une représentation (50) susceptible d'être animée supplémentaire dans le système d'affichage (35), laquelle comprend au moins une partie de la première unité graphique (29), l'au moins une partie de la première unité graphique (29) étant mise en évidence avec ses caractéristiques géométriques, de préférence la représentation (50) susceptible d'être animée supplémentaire pouvant représenter une mise en évidence colorée de la paroi pulmonaire (53) du poumon et / ou une restriction de la trachée (48) étant susceptible d'être représentée de manière animée et / ou la diaphragme (55) étant susceptible d'être représenté dans une couleur distinguible.
